# EUROPEAN PATENT APPLICATION

(11) **EP 1 462 097 A1**
(43) Date of publication of application: **29.09.2004**
(21) Application number: 04007039.3
(22) Date of filing: 24.03.2004
(51) Int. Cl.: A61K 9/16, A61K 9/50

(54) **Process for the preparation of enteric-coated and /or controlled-release medicaments containing active principles sensitive to gastrointestinal environment**

(30) Priority: 28.03.2003 IT MI20030616
(71) Applicant: Acme Drugs S.r.l., 42025 Cavriago (Emilia) (IT)
(72) Inventor: Predieri, Giulio, 42025 Cavriago (IT)
(74) Representative: Minoja, Fabrizio, Dr.

(57) **Abstract**

Veterinary, dietetic or pharmaceutical compositions containing active principles sensitive to the human and animal gastrointestinal environment and precess for the preparation thereof.

## Description

### Field of the invention

The present invention relates to a process for the preparation of products for the oral use containing active principles sensitive to the Human and animal gastrointestinal environment, and/or degradable in acidic media (for example rumen in the case of ruminants), in particular medicaments containing omeprazole, or other proton pump inhibitors suitable for the treatment of gastric ulcer in men and animals.

The invention further relates to enteric-coated granulates obtainable by said process,'suitable for the preparation of medicaments, dietetics, functional foodstuffs, nutraceuticals, probiotics, feed premixtures and excipients for zootechnical feeding.

### BACKGROUND OF THE INVENTION

Coating of granulates or active principles with various substances (acrylic polymers, amides, cellulose and cellulose-derivatives, fats etc.), to provide gastroprotection or controlled-release of the active principles has been known for some time.

Nevertheless, this technique has not been applied to a particular class of antisecretory drugs, known as proton pump inhibitors, for example omeprazole, lansoprazole and pantoprazole, characterized by high instability towards acids.

For these medicaments, in particular omeprazole, enteric-coated formulations in the form of granulates for the oral use have been proposed; the granulates can be obtained through a process comprising three main productive phases:
Phase I: production of neutral pellets (for example dextrose, saccharose, starch beads) suitable as carriers for active principles. Neutral pellets have a spheroidal shape and a smooth surface in order to allow even adhesion of the active principle in the following phases.
Phase II (enrichment): addition of the active principle to the pellets. During this phase the pellets are coated with one or more layers of active principle until the amount comprised in the final medicament is reached. This phase requires very long times, further to troublesome and expensive controls. The active principle can be added in the liquid form (with a spray system), or as a powder by means of different dispensers.
Phase III (coating): coating of the product from phases I and II with various gastroprotective substances, depending on the desired results. During this phase the pellets "embedded" in the active ingredient are coated with one layer of gastroenteric film-agent. Industrial production may require intermediate work-up phases such as recovery of the granules by means of insulating materials or ligands (for example example talc, cellulose) and/or opacifiers (titanium dioxide).

This process is expensive and difficult to reduce to practice, as it comprises different critical phases that migh jeopardise the quality of the final product.

The economic aspect is of utmost importance in the veterinary and zootechnical field. For example, the aforementioned proton pump inhibitors have been proposed for veterinary uses, for example for improving the performances of race horses affected by gastric ulcer. Nevertheless, gastric ulcer therapy in horses with conventional medicaments containing omeprazole is still unaffordable for most of the owners, as it requires protracted treatments (20-30 days of consecutive administration) and a considerable amounts of drug, in view of the weight of the animal.

Therefore, in the veterinary field there is still a urgent need for stable medicaments suitable for oral administration, that can be formulated in an easy way and at low cost.

### DISCLOSURE OF THE INVENTION

Accordingly, the invention provides a process for direct enteric coating which allows to prepare granulates containing active principles sensitive to acidic media or degradable in the gastrointestinal environment, in a simpler and cheaper way than the processes known in the art.

In particulare, the process of the invention allows to obtain enteric-coated granules without the preparation of neutral pellets and coating-pan enrichment (i.e. phases I and II).

The process of the invention comprises the following steps:
A) homogeneous mixing of the active principles with granulation excipients, followed by dry granulation and calibration;
B) spheronization of the granules;
C) coating with a gastroprotective polymer.

In phase A, mixing is carried out on raw materials in the form of powders, preferably by means of V-mixers of triple capacity compared with the volume of powders to mix. The mixing process gives good results when carried out on powders having omogeneous particle size and for times ranging from 5 to 15 minutes.

The mixture of powders comprises up to 40% by weight of the active ingredient (for example omeprazole, lansoprazole or lantoprazole), a diluent, a lubricant and glidant.

Examples of diluents are lactose, starch, monohydrate bicalcium phosphate, microcrystalline cellulose, preferably lactose, in amount of about 58.5% by weight.

Examples of suitable lubricants are magnesium stearate, polyethylene glycols, metal stearates, preferably magnesium stearate in amount of about 0.5% by weight.

A suitable glidant is colloidal silica, in amount of about 1% weight.

Granulation is carried out under dry conditions, directly on the mixed powders, preferably by means of a "barrel mill" which produces slugs of compacted powder or thin layers that are subsequently ground by means of a granulator.

The resulting granulate is sieved through a 1 mm mesh sieve.

Sieved granules surface is still too uneven to be coated with a gastroprotective film of homogeneous thickness.

Lack of homogeneity in the thickness of the proctetive layer and concomitant breaks in the surface make it difficult to achieve satisfactory gastroprotection by coating and therefore it is necessary, in order to improve the uniformity of the protective layer, to submit the granules to spheronization.

This operation is accomplished by means of a specific equipment (spheronizator), consisting of a rotating grained steel plate which acts on the granules as a microwheel, thus making their surface regular.

The process allows to apply on the granules surface a homogeneous coating and at the same time reduces the amount of gastroprotective film.

Spheronized granules are then placed in coating-pan and spray-coated with Eudragit L30D55. The coating film amounts to about 12% by weight of the final product.

The resulting granules can be incorporated in a paste, in a gel or in a fat paste, tablets, capsules or syrups and administered to horses or other animals for the therapy duodenal ulcer.

The process of the invention is considerably advantageous from the economic standpoint, because it does not require the use neutral pellets considerably shortens the permanence time of the granulate in the coating-pan, reduces error probability in dosing the active principle and degradation of the active ingredient.

Moreover, the process can be carried out on powdery raw material, which renders unnecessary hot air drying of semifactured products and considerably reduces the risk of oxidation of the active principle.

The following example discloses the invention in more detail.

### EXAMPLE

### Omeprazole coated granules

Composition for 100 g:
active ingredient: omeprazole g 28.30.
excipients: lactose D 80 g 58.38, Aerosil 200 (anhydrous colloidal silica) g 0.88, magnesium stearate g 0.44, Eudragit L30D55 g 12.00.

The medicament obtainable by to the process of the invention ensures protection of the active ingredient from acidic media; more than 90% of the active ingredient remains unchanged after 60 minutes at pH 2.

| **Omeprazole Release Curve in a solution** **buffered to pH 2** | | | | | |
|---|---|---|---|---|---|
| granules omeprazole + eudragit 12% | | omeprazole | | uncoated omeprazole granules | |
| **TIME** **(hours)** | **% dissolved Omeprazole** | **TIME** **(hours)** | **% dissolved Omeprazole** | **TIME** **(hours)** | **% dissolved Omeprazole** |
| 0.00 | 0.00% | 0.00 | 0 | 0.00 | 0 |
| 1.00 | 6.50% | 0.20 | 18.28% | 0.30 | 64.60% |
| 3.30 | 21.00% | 0.40 | 59.76% | 1.00 | 88.30% |
| 5.00 | 28.70% | 1.00 | 100.00% | | |
| 7.00 | 33.60% | | | | |

The formulation fully meets therapeutical needs, as also demonstrated by clinical tests on horses carried out according to European Good Clinical Practices.

The tests confermed that the medicament is effective, safe, and easy to use in the therapy of gastric ulcer in horses.

## Claims

1. A process for the preparation of products for the oral use containing active principles sensitive to gastrointestinal environment, which comprises the following steps:
A) homogeneous mixing of the active principles with granulation excipients, followed by dry granulation and calibration;
B) spheronization of the granules;
C) spray coating with a gastroprotective polymer.

2. Process as claimed in claim 1 wherein the active principles are selected from omeprazole, lansoprazole, pantoprazole.

3. Process as claimed in claim 2 wherein the active ingredient is omeprazole.

4. Process as claimed in any one of claims 1 to 3, wherein phase A comprises mixing powders that contain up to 40% by weight of the active ingredient, a diluent, a lubricant and a glidant.

5. Process as claimed in claim 4 wherein the diluents are selected from lactose, starch, monohydrate bicalcium phosphate, microcrystalline cellulose.

6. Process as claimed in claim 4 wherein the lubricants are selected from magnesium stearate, polyethylene glycols, metal stearates.

7. Process as claimed in claim 4 wherein the glidant is colloidal silica.

8. Process as claimed in claim 4 wherein the diluent is lactose in amount of up to 58.5% by weight, the lubricant is magnesium stearate in amount of 0.5% by weight and colloidal silica is present in amount of 1% by weight.

9. Process as claimed in any one of claims from 1 to 8 wherein the granulation phase is carried under dry conditions, directly on the mixed powders.

10. Process as claimed in any one of claims from 1 to 9 wherein the granulates are coated with Eudragit L30D55.

11. Enteric coated granulates obtainable by the process of claims 1-10.

12. Pharmaceutical or veterinary compositions and feed premixtures comprising the granulates of claim 11 in a form suitable for the oral administration.

13. Compositions as claimed in claim 12 in the form of pastes, gels, tablets, capsules, syrups.
